# EUROPEAN PATENT APPLICATION

(11) **EP 4 501 388 A1**
(43) Date of publication of application: **05.02.2025**
(21) Application number: 23780679.9
(22) Date of filing: 29.03.2023
(51) Int. Cl.: A61M 25/10, A61B 18/14

(54) **BALLOON CATHETER AND BALLOON CATHETER SYSTEM**

(30) Priority: 29.03.2022 JP 2022053119
(71) Applicant: Toray Industries, Inc., Tokyo 103-8666 (JP)
(72) Inventor: TSUKAMOTO, Kota, Tokyo 103-8666 (JP); HARADA, Hiroyuki, Tokyo 103-8666 (JP); TAKAOKA, Motoki, Osaka-shi, Osaka 530-8222 (JP)
(74) Representative: Kador & Partner Part mbB
(86) International application number: PCT/JP2023/012770
(87) International publication number: WO 2023/190670

(57) **Abstract**

The present invention aims to provide a balloon catheter and a balloon catheter system capable of safely keeping the surface temperature of a balloon constant. A balloon catheter according to the present invention includes: a balloon; an outer cylinder shaft connected to a proximal end of the balloon; an inner cylinder shaft passing through the outer cylinder shaft and connected to a distal end of the balloon; a heating member for heating a liquid in the balloon; a first temperature sensor fixed to an end of the heating member; and a liquid passage formed between the outer cylinder shaft and the inner cylinder shaft and leading into the balloon. The balloon can be stretched or loosened by relative movement of the inner cylinder shaft to the outer cylinder shaft, and the heating member is arranged in the balloon and fixed only to the outer cylinder shaft.

## Description

### TECHNICAL FIELD

The present invention relates to a balloon catheter and a balloon catheter system.

### BACKGROUND ART

Catheter ablation treatment is a treatment method for ablating a target site in a body by using a catheter inserted in the body. As an example, medical conditions such as arrhythmia caused by atrial fibrillation, endometriosis, and cancer are treated by destroying the target sites by ablation. As the catheter used for the catheter ablation treatment, a balloon catheter having a balloon at the distal end is known as disclosed in Patent Document 1 and Patent Document 2.

When the balloon catheter is inserted into the body, the balloon is deflated and stretched in a longitudinal direction of the balloon catheter. Then, a liquid is injected into the balloon catheter inserted in the body, and the balloon is inflated. Since a temperature of the liquid in the balloon is adjusted, a surface temperature of the balloon can be controlled. The balloon adjusted to a predetermined surface temperature is brought into contact with a circumferential target site, such as a site of a vein connected to the atrium, thereby allowing to ablate the circumferential target site in one step.

### PRIOR ART DOCUMENTS

### PATENT DOCUMENTS

[Patent Document 1] JP 3611799 B
[Patent Document 2] JP 4747141 B
[Patent Document 3] WO2021/201078

### SUMMARY OF THE INVENTION

### PROBLEMS TO BE SOLVED BY THE INVENTION

For the treatment using the balloon catheter, it is important to accurately grasp the surface temperature of the balloon and safely obtain the constant surface temperature thereof. In this regard, the balloon catheters disclosed in Patent Document 1 and Patent Document 2 are each provided with a temperature sensor for measuring the surface temperature of the balloon. In Patent Document 1, the temperature sensor attached to an inner surface of the balloon is used. However, it is not easy to stably install the temperature sensor on the surface of the balloon, which is inflated from a deflated state. In this regard, in Patent Document 2, it is proposed to employ a two-layer structure for the balloon and arrange the temperature sensor between layers. IIowever, the balloon catheter in Patent Document 2 has not been widely used due to difficulties in actual production in terms of manufacturing of the balloon, installation of a heat-sensitive section of the temperature sensor, handling of a lead wire in the temperature sensor, and the like. In other words, in the conventional balloon catheters, it is difficult to identify the surface temperature of the balloon with a high degree of accuracy.

In Patent Document 3, a catheter in which the surface temperature of the balloon is measured with a high degree of accuracy by arranging the temperature sensor in a liquid passage of the balloon catheter is proposed. However, such a balloon catheter shows a significant change in the balloon surface temperature, depending on a contact state between the balloon and a target object. Thus, it is difficult to keep the balloon surface temperature constant. Furthermore, in Patent Document 3, a method for controlling the balloon surface temperature by using the temperature sensor arranged in the liquid passage of the balloon catheter is described. However, when a temperature discrepancy between a heating member arranged in the balloon and the balloon surface becomes significant, it is difficult to obtain the desired balloon surface temperature from a safety perspective.

The present invention has been made in view of the points described so far and aims to provide a balloon catheter and a balloon catheter system capable of safely keeping a balloon surface temperature constant.

### MEANS FOR SOLVING THE PROBLEMS

In order to solve the above problem, the present inventors intensively studied to discover the following inventions (1) to (3):
(1) A balloon catheter, comprising:
   a balloon;
   an outer cylinder shaft connected to a proximal end of the balloon;
   an inner cylinder shaft passing through the outer cylinder shaft and connected to a distal end of the balloon;
   a heating member for heating a liquid in the balloon;
   a first temperature sensor fixed to an end of the heating member; and
   a liquid passage formed between the outer cylinder shaft and the inner cylinder shaft and leading into the balloon,
   wherein the balloon can be stretched or loosened by relative movement of the inner cylinder shaft to the outer cylinder shaft, and
   the heating member is arranged in the balloon and fixed only to the outer cylinder shaft.
(2) The balloon catheter of (1),
   wherein the outer cylinder shaft has an extending section extending into the balloon, and
   the heating member is fixed to the extending section.
(3) A balloon catheter system, comprising:
   the balloon catheter of (1) or (2);
   a supplier for supplying the liquid to the liquid passage;
   an agitator for agitating the liquid in the balloon by repeatedly supplying the liquid to the liquid passage and discharging the liquid from the liquid passage; and
   a heater electrically connected to the heating member and applying electrical energy to the heating member,
   wherein the heater applies the electrical energy to the heating member based on information on a temperature acquired by the first temperature sensor.

### EFFECT OF THE INVENTION

According to the present invention, the surface temperature of the balloon can be safely kept constant.

### BRIEF DESCRIPTION OF THE DRAWINGS

Fig. 1 is a view for illustrating a first embodiment and illustrating a balloon catheter system and a balloon catheter.
Fig. 2 is a view illustrating a distal end portion of the balloon catheter in Fig. 1 in an inflated state of a balloon.
Fig. 3 is a view illustrating the distal end portion of the balloon catheter in Fig. 1 in a deflated and stretched state of the balloon.
Fig. 4 is a cross-sectional view taken along line IV-IV in Fig. 2.
Fig. 5 is a cross-sectional view taken along line V-V in Fig. 2.
Fig. 6 is a view illustrating a second embodiment and illustrating the distal end portion of the balloon catheter in Fig. 1 in the inflated state of the balloon.
Fig. 7 is a view illustrating a third embodiment and illustrating the distal end portion of the balloon catheter in Fig. 1 in the inflated state of the balloon.
Fig. 8 is a view illustrating a balloon catheter in which a heating member is not fixed to an outer cylinder shaft in a comparative example.
Fig. 9 illustrates temperature distribution in the distal end portion of the balloon catheter in a coaxial state, which is acquired by a thermo-fluid analysis using CAE.
Fig. 10 illustrates the temperature distribution in the distal end portion of the balloon catheter in a non-coaxial state, which is acquired by the thermo-fluid analysis using the CAE.
Fig. 11 is a view illustrating an experimental method using the balloon catheter system.
Fig. 12 is a view illustrating the distal end portion of the balloon catheter and illustrating a state where a catheter is pressed hard against a treatment site.
Fig. 13 is a graph illustrating measurement data of differences between a temperature of the heating member and a balloon surface temperature.
Fig. 14 is a view illustrating the distal end portion of the balloon catheter and illustrating a flow of a liquid when the liquid is discharged from a liquid passage into the balloon in the coaxial state.
Fig. 15 is a view illustrating the distal end portion of the balloon catheter and illustrating the flow of the liquid when the liquid is suctioned from the inside of the balloon to the liquid passage in the coaxial state.
Fig. 16 is a view illustrating the distal end portion of the balloon catheter and illustrating the flow of the liquid when the liquid is discharged from the liquid passage into the balloon in the non-coaxial state.
Fig. 17 is a view illustrating the distal end portion of the balloon catheter and illustrating the flow of the liquid when the liquid is suctioned from the inside of the balloon to the liquid passage in the non-coaxial state.
Fig. 18 is a view illustrating the distal end portion of the balloon catheter in the comparative example and illustrating the flow of the liquid when the liquid is suctioned from the inside of the balloon to the liquid passage in the coaxial state.
Fig. 19 is a view illustrating the distal end portion of the balloon catheter in the comparative example and illustrating the flow of the liquid when the liquid is suctioned from the inside of the balloon to the liquid passage in the coaxial state.
Fig. 20 is a view illustrating the distal end portion of the balloon catheter in the comparative example and illustrating the flow of the liquid when the liquid is discharged from the liquid passage into the balloon in the non-coaxial state.
Fig. 21 is a view illustrating the distal end portion of the balloon catheter in the comparative example and illustrating the flow of the liquid when the liquid is suctioned from the inside of the balloon to the liquid passage in the non-coaxial state.

### MODE FOR CARRYING OUT THE INVENTION

Hereinafter, an embodiment of the present invention is described with reference to specific examples illustrated in the drawings. Terms such as "parallel", "orthogonal", and "identical" and values such as of lengths and angles used in this specification to specify shapes, geometric conditions, and degrees thereof are not bound to strict meanings, but are interpreted to include a range within which similar functions can be expected.

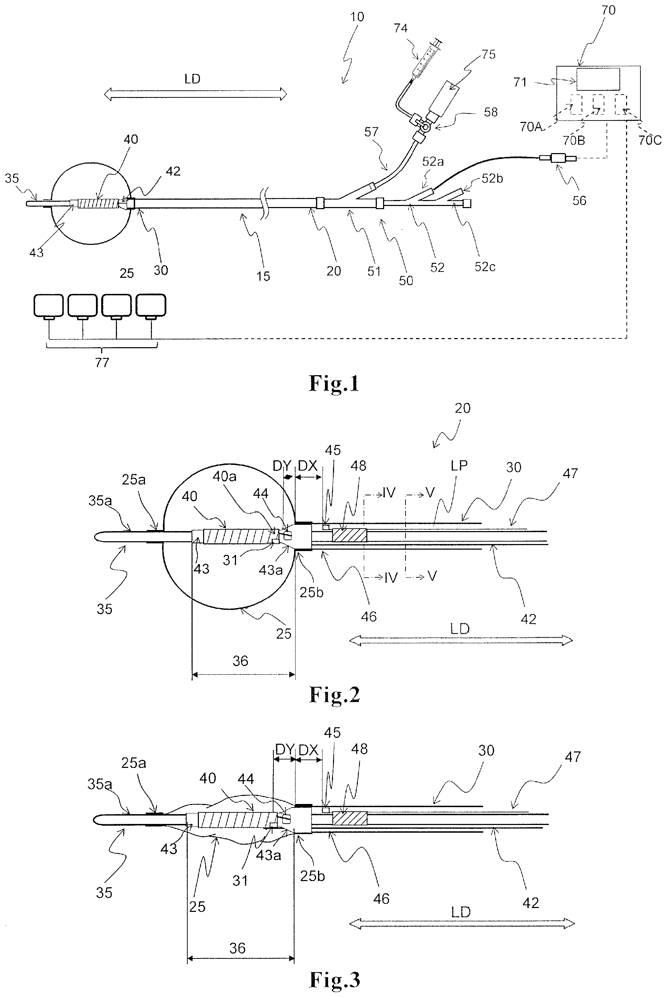

A balloon catheter system 10 according to a first embodiment illustrated in Fig. 1 has a balloon catheter 15 as well as a controller 70 and an agitator 75, each of which is connected to the balloon catheter 15. The balloon catheter 15 has a catheter body 20 having a longitudinal direction LD and a handle 50 connected to the proximal end of the catheter body 20.

The catheter body 20 illustrated in Fig. 2 has a balloon 25, an outer cylinder shaft 30 connected to the proximal end 25b of the balloon 25, an inner cylinder shaft 35 connected to the distal end 25a of the balloon 25, a heating member 40 arranged in the balloon 25, and a first temperature sensor 31 connected to the proximal end 40a of the heating member 40. The inner cylinder shaft 35 passes through the outer cylinder shaft 30 and extends into the balloon 25. A liquid passage LP leading into the balloon 25 is formed on a proximal side of the inside of the balloon 25 and between the outer cylinder shaft 30 and the inner cylinder shaft 35. The heating member 40 heats a liquid in the balloon 25.

The catheter body 20 can identify a surface temperature of the balloon 25, into which the heated liquid is injected, with a high degree of accuracy by using a second temperature sensor 45 installed in the liquid passage LP. In particular, the catheter body 20 according to this embodiment is devised to keep the surface temperature of the balloon 25, which is identified by using the second temperature sensor 45, constant. More specifically, by fixing the outer cylinder shaft 30 and the heating member 40, the heating member 40 and the balloon 25 are installed such that a length DY between the heating member 40 and the proximal end 25b of the balloon 25 is always constant, and a positional relationship between the heating member 40 and a hole 44 formed on a tapered transition section 43a provided in a fixed member 43 becomes constant, thereby allowing to keep the temperature detected by the first temperature sensor 31, which is installed at the proximal end 40a of the heating member, and the temperature detected by the second temperature sensor, which is installed in the liquid passage LP to identify the surface temperature of the balloon 25, constant.

The longitudinal direction LD of the catheter body 20 is identified as a direction in which center axes of the outer cylinder shaft 30 and the inner cylinder shaft 35 extending from the outer cylinder shaft 30 extend. In this specification, the "distal" side used for each component of the balloon catheter 15 and the catheter body 20 means a side that is away from the handle 50 and an operator (practitioner) of the balloon catheter 15 along the longitudinal direction LD of the catheter body 20, in other words, a tip side. The "proximal" side used for each component of the balloon catheter 15 and the catheter body 20 means a side close to the handle 50 and the operator (practitioner) of the balloon catheter 15 along the longitudinal direction LD of the catheter body 20, in other words, a proximal end side.

Hereinafter, the balloon catheter system 10 and the balloon catheter 15 are described in detail. First, the catheter body 20 constituting the balloon catheter 15 is described in detail. As described above, the catheter body 20 constituting the balloon catheter 15 according to this embodiment has the balloon 25, the outer cylinder shaft 30, the inner cylinder shaft 35, the heating member 40, the first temperature sensor 31, and the second temperature sensor 45.

Of these, both the outer cylinder shaft 30 and the inner cylinder shaft 35 are formed in tubular shapes, typically, cylindrical shapes. Thus, each of the outer cylinder shaft 30 and the inner cylinder shaft 35 is formed with a lumen as an internal space. For example, a guide wire, which is not illustrated, can be inserted into the lumen formed by the inner cylinder shaft 35. The inner cylinder shaft 35 is inserted into the lumen formed by the outer cylinder shaft 30. In other words, the outer cylinder shaft 30 and the inner cylinder shaft 35 constitute a configuration of a double-tube shaft. The inner diameter of the outer cylinder shaft 30 is larger than the outer diameter of the inner cylinder shaft 35. Thus, the lumen remains between the outer cylinder shaft 30 and the inner cylinder shaft 35. This lumen between the outer cylinder shaft 30 and the inner cylinder shaft 35 forms the liquid passage LP. As illustrated in Fig. 2, the liquid passage LP leads into the balloon 25. In addition, the liquid passage LP extends into the handle 50.

The length of the outer cylinder shaft 30 is preferably 500 mm or longer and 1700 mm or shorter and further preferably 600 mm or longer and 1200 mm or shorter. The length of the inner cylinder shaft 35 is preferably 500 mm or longer and 1700 mm or shorter and further preferably 600 mm or longer and 1200 mm or shorter. By making the outer cylinder shaft 30 shorter than the inner cylinder shaft 35 by 0 mm to 40 mm, the outer cylinder shaft 30 can be used as an extending section that extends into the balloon 25.

Each of the outer cylinder shaft 30 and the inner cylinder shaft 35 is preferably fabricated by using a flexible material having an excellent antithrombotic property. Examples of the flexible material having the excellent antithrombotic property include, but are not limited to, fluoropolymers, polyamides, polyurethane-based polymers, and polyamides. In addition, the outer cylinder shaft 30 is preferably fabricated by laminating layers of different flexible materials in order to simultaneously ensure slidability against the inner cylinder shaft 35 and adhesiveness or heat-weldability to the balloon 25. Here, the outer diameter of the inner cylinder shaft 35 is preferably 1.4 mm or longer and 1.7 mm or shorter. The inner diameter of the inner cylinder shaft 35 is preferably 1.1 mm or longer and 1.3 mm or shorter.

The balloon 25 is connected to a side surface of the outer cylinder shaft 30 and a tip of the inner cylinder shaft 35. The balloon 25 is formed to be inflatable by injecting the liquid and deflatable by discharging the liquid. The balloon 25 preferably has a shape capable of fitting a target site (for example, a blood vessel) as a treatment target. As an example, a spherical shape with a diameter of 15 mm or longer and 40 mm or shorter can be employed as the shape of the balloon 25 that matches a pulmonary venous junction of the left atrium. Examples of the spherical shape include a true spherical shape, an oblate spherical shape, a prolate spherical shape, and a substantially spherical shape.

The wall thickness of the balloon 25 is preferably 10 µm or greater and 200 µm or less. In addition, the material for the balloon 25 is preferably an elastic material with an excellent antithrombotic property and, more specifically, a polyurethane-based polymer material or the like can be used. Examples of the polyurethane-based polymer material employed for the balloon 25 include thermoplastic polyether urethane, polyether polyurethane urea, fluorinated polyether urethane urea, polyether polyurethane urea resin, and polyether polyurethane urea amide.

As illustrated in Fig. 2 and Fig. 3, in the illustrated catheter body 20, the distal end (tip) 25a of the balloon 25 is fixed to the distal end (tip) 35a of the inner cylinder shaft 35. The proximal end (proximal end) 25b of the balloon 25 is fixed to the side surface of the outer cylinder shaft 30. The balloon 25 can be joined to the outer cylinder shaft 30 and the inner cylinder shaft 35 by adhesion or heat welding.

In a first embodiment, the proximal end (proximal end) 25b of the balloon 25 is fixed to the side surface of the outer cylinder shaft 30, beyond which the outer cylinder shaft 30 extends into the balloon 25 to form an extending section 36 of the outer cylinder shaft 30. Here, the extending section refers to a portion of the outer cylinder shaft that extends into the balloon.

As illustrated in Fig. 2 and Fig. 3, the outer cylinder shaft 30 in the first embodiment may have the extending section 36 as a separate component. In this case, the outer cylinder shaft 30 is composed of a tubular intermediate member 46 and a fixed member 43 fixed to the distal end (tip) of the tubular intermediate member 46, and the fixed member 43 forms the extending section 36 of the outer cylinder shaft 30. In another embodiment, the outer cylinder shaft 30 may be formed as a single-piece shaft.

Here, the outer diameter of the tubular intermediate member 46 is preferably 3.0 mm or larger and 4.0 mm or smaller, and the inner diameter of the tubular intermediate member 46 is preferably 2.5 mm or larger and 3.5 mm or smaller.

The fixed member 43 illustrated in Fig. 2 and Fig. 3 has a tubular section 43b having a large diameter on the proximal side, a tubular section 43c having a small diameter on the distal side, and the tapered transition section 43a therebetween. The tapered transition section 43a connects the tubular section having the large diameter and the tubular section having the small diameter.

In the fixed member 43, the outer diameter of the tubular section 43b having the large diameter, which is a portion connected to the tubular intermediate member 46, is preferably 3.0 mm or larger and 4.0 mm or smaller, and the inner diameter of the tubular section 43b having the large diameter is preferably 2.5 mm or larger and 3.5 mm or smaller. The outer diameter of the tubular section 43c having the small diameter, to which the heating member 40 is fixed, is preferably 1.5 mm or larger and 3.0 mm or smaller, and the inner diameter of the tubular section 43c having the small diameter is preferably 1.5 mm or larger and 2.0 mm or smaller. Furthermore, the tapered transition section 43a preferably has one or more holes 44, through each of which a conductive wire runs and which serves as the liquid passage, and the diameter of the hole 44 is preferably 0.5 mm² or larger and 2 mm² or smaller.

Relative movement of the outer cylinder shaft 30 and the inner cylinder shaft 35 in the longitudinal direction LD causes deformation of the balloon 25 connected to the outer cylinder shaft 30 and the inner cylinder shaft 35. The relative movement of the outer cylinder shaft 30 to the inner cylinder shaft 35 allows to stretch or loosen the balloon 25, thereby allowing to adjust the dimension of the balloon 25 in the longitudinal direction LD. Here, "to stretch" refers to a state where the width of the balloon 25 in the longitudinal direction LD is increased and a tensile force is applied to the balloon 25, while "to loosen" refers to a state where a tensile force is not applied to the balloon 25.

As illustrated in Fig. 3, when the inner cylinder shaft 35 moves relative to the outer cylinder shaft 30 toward the distal side in the longitudinal direction LD, the balloon 25 is stretched in the longitudinal direction LD and brought into a further tense state. In the illustrated example, a movement range of the inner cylinder shaft 35 relative to the outer cylinder shaft 30 toward the distal side in the longitudinal direction LD is limited by the balloon 25. When the inner cylinder shaft 35 moves relative to the outer cylinder shaft 30 toward the proximal side in the longitudinal direction LD from the state illustrated in Fig. 3, the balloon 25 is brought into a loosened state.

Furthermore, by injecting the liquid into the loosened balloon 25, as illustrated in Fig. 2, the balloon 25 can be inflated, and the dimension of the balloon 25 in the short direction can be adjusted.

Next, the heating member 40 is described. The heating member 40 is arranged in the balloon 25 and fixed only to the outer cylinder shaft 30. The heating member 40 is a member for heating the liquid injected into the balloon 25.

The heating member 40 only needs to be arranged in the balloon 25 and fixed only to the outer cylinder shaft 30. More specifically, as illustrated in Fig. 2 and Fig. 3, the heating member 40 is formed on the extending section 36 connected to the balloon 25, in the outer cylinder shaft 30 and provided in a manner to surround the inner cylinder shaft 35 extending into the balloon 25.

As another embodiment, the outer cylinder shaft 30 that does not have the extending section 36 extending into the balloon 25 may be used. In this case, the outer cylinder shaft 30 does not have the extending section 36, and the proximal end of the balloon 25 is connected to the distal end of the outer cylinder shaft 30. Thus, the outer cylinder shaft 30 does not enter the balloon 25. Meanwhile, the proximal end of the heating member 40 is fixed only to the distal end of the outer cylinder shaft 30, and is provided in the manner to surround the inner cylinder shaft 35 extending into the balloon 25. At this time, the outer cylinder shaft 30 and the heating member 40 can be fixed by using a technique such as adhesion or welding.

As an example of the heating member 40, a nichrome wire generating electric resistance heat can be selected. As another example of the heating member 40, as illustrated in Fig. 2 and Fig. 3, a coil electrode can be selected. With high-frequency energization of the heating member 40, a high-frequency current flows between the heating member 40 and a counter electrode 77 (Fig. 1) arranged outside, causing the liquid located between the heating member 40 and the counter electrode 77 to generate Joule-heat. The counter electrode 77 is arranged on the back of a patient, for example.

In the example illustrated in Fig. 2 and Fig. 3, the heating member 40 is formed on the extending section 36 connected to the balloon 25, in the outer cylinder shaft 30 and provided in the manner to surround the inner cylinder shaft 35 extending into the balloon 25. The heating member 40 configured by the coil electrode can be formed by the wound conductive wire. The heating member 40 is electrically connected to a wire 42 for the high-frequency energization. The wire 42 extends to the handle 50 through the liquid passage LP as the lumen between the outer cylinder shaft 30 and the inner cylinder shaft 35. As a specific example of the coil electrode, a lead wire used for the wire 42 can be used after peeling the insulating coating off and winding around a mandrel, such that the lead wire is connected to the outer cylinder shaft 30, and arranged in the manner to surround the inner cylinder shaft 35. Since such a coil electrode is integrally formed with the wire 42, such a coil electrode can effectively suppress occurrence of defect such as wire breakage.

In the example illustrated in Fig. 2, the outer cylinder shaft 30 is composed of the tubular intermediate member 46 and the fixed member 43 fixed to the distal end (tip) of the tubular intermediate member 46. The tapered transition section 43a in the fixed member 43 has the hole 44 through which the conductive wire runs and which serves as the liquid passage. The coil electrode is formed of the conductive wire wound around the fixed member 43.

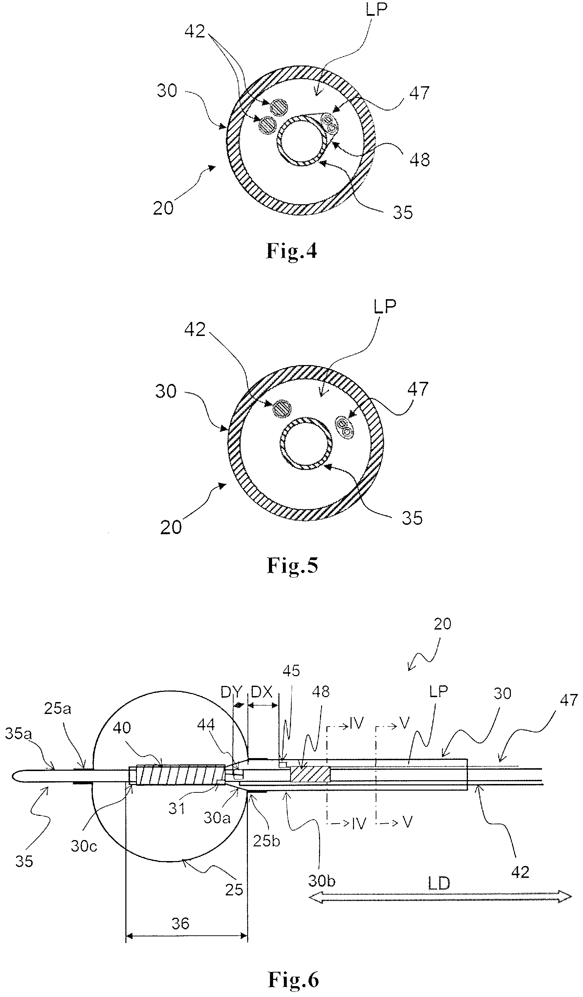

Diameters of the coil electrode and the wire 42 are preferably 0.1 mm or larger and 1 mm or smaller and further preferably 0.1 mm or larger and 0.4 mm or smaller. Examples of the conductive material for each of the coil electrode and the wire 42 include copper, silver, gold, platinum, and alloys thereof. In order to prevent a short circuit, the wire 42 preferably has a configuration that the conductive wire made of the conductive material is coated with an insulating coating such as a fluoropolymer (see Fig. 4 and Fig. 5).

The first temperature sensor 31 is installed at the proximal end of the heating member 40 composed of the coil electrode, and the first temperature sensor 31 obtains the temperature of the heating member 40. The safer treatment can be performed by managing and controlling the temperature of the heating member 40 which is a component having the highest temperature of the balloon catheter.

Next, the second temperature sensor 45 is described. The second temperature sensor 45 obtains information on the temperature of the liquid. In this embodiment, the second temperature sensor 45 is arranged in the liquid passage LP located on the proximal end side of the balloon 25 and between the outer cylinder shaft 30 and the inner cylinder shaft 35. The information on the liquid temperature in the liquid passage LP can be acquired by the second temperature sensor 45.

According to the study by the present inventors, based on the information acquired by this second temperature sensor 45, the surface temperature of the balloon 25, which is important for the ablation treatment using the balloon catheter system 10, can be identified with the high degree of accuracy. The installation of the second temperature sensor 45 in the liquid passage LP significantly facilitates manufacturing of the catheter body 20 when compared to the installation of the second temperature sensor 45 in the balloon 25.

Furthermore, when compared to the installation of the second temperature sensor 45 in the balloon 25, the installation of the second temperature sensor 45 in the liquid passage LP enables protection of the second temperature sensor 45 against external stress and stable support thereof. That is, by installing the second temperature sensor 45 in the liquid passage LP, it is possible to largely improve quality and reliability of the balloon catheter system 10 and the balloon catheter 15.

For the purpose of identifying the surface temperature of the balloon 25 with the high degree of accuracy, strictly speaking, the length DX between the proximal end 25b of the balloon 25 and the second temperature sensor 45 in the longitudinal direction LD depends on liquid supply/discharge amounts by the agitator 75 described below. However, in consideration of each dimension of the catheter body 20, which is normally used for cardiac ablation treatment, and the liquid supply/discharge amounts by the agitator 75, the length DX (see Fig. 2) between the proximal end 25b of the balloon 25 and the second temperature sensor 45 is preferably 5 mm or longer and 150 mm or shorter, and further preferably 10 mm or longer and 20 mm or shorter.

Unless otherwise specifically described, the length DX between the proximal end 25b of the balloon 25 and the second temperature sensor 45 is a length identified in a state illustrated in Fig. 2 where the balloon 25 is inflated with the liquid.

A thermocouple or a thermistor can be used as each of the first temperature sensor 31 and the second temperature sensor 45. In addition, a T-type thermocouple is particularly suited as each of the first temperature sensor 31 and the second temperature sensor 45. Use of the T-type thermocouple can reduce heat capacity of a heat-sensitive section. Furthermore, by selecting the T-type thermocouple as each of the first temperature sensor 31 and the second temperature sensor 45, a thermoelectromotive force is stabilized. Moreover, since a temperature range from 50 °C to 80 °C can be detected with a high degree of accuracy, a T-type thermocouple is particularly suited for the cardiac ablation treatment. Here, the information on the temperature acquired by each of the first temperature sensor 31 and the second temperature sensor 45 is an electric potential that can be acquired from the thermocouple or a resistance value that can be acquired from the thermistor, for example.

As illustrated in Fig. 2 and Fig. 3, each of the first temperature sensor 31 and the second temperature sensor 45 typically has the heat-sensitive section and a lead wire 47 electrically connected to the heat-sensitive section. In the case of a thermocouple, the portion connected with a different type of metal forms the heat-sensitive section in each of the first temperature sensor 31 and the second temperature sensor 45. In the case of a thermistor, a ceramic element forms the heat-sensitive section in each of the first temperature sensor 31 and the second temperature sensor 45. The lead wire 47 extends to the handle 50 through the liquid passage LP as the lumen between the outer cylinder shaft 30 and the inner cylinder shaft 35.

The diameter of the lead wire 47 is preferably 0.05 mm or larger and 0.5 mm or smaller, and further preferably 0.05 mm or larger and 0.3 mm or smaller. In the first temperature sensor 31 and the second temperature sensor 45 as the thermocouples, for example, copper can be used for the one lead wire 47 while constantan can be used for the other lead wire 47. In order to prevent the short circuit of the paired lead wires 47, as illustrated in Fig. 4 and Fig. 5, each of the paired lead wires 47 preferably has electrically insulating coating such as a fluoropolymer or enamel.

As illustrated in Figs. 2 to 4, since the lead wire 47 is fixed by using fixing means 48, the second temperature sensor 45 is fixed to the inner cylinder shaft 35 via the lead wire 47. In addition, the second temperature sensor 45 is located away from both the outer cylinder shaft 30 and the inner cylinder shaft 35. In other words, the second temperature sensor 45 is not in contact with the outer cylinder shaft 30 and the inner cylinder shaft 35. Thus, it is possible to avoid degraded responsiveness of the second temperature sensor 45, which is caused by the temperatures of the outer cylinder shaft 30 and the inner cylinder shaft 35 which has a large heat capacity. As a result, the liquid temperature in the liquid passage LP can be evaluated with a high responsiveness and a high degree of accuracy by using the second temperature sensor 45.

Various types of means can be used as the fixing means 48 for fixing the lead wire 47 to the inner cylinder shaft 35 without any particular limitation. In the illustrated example, a heat-shrinkable tube that shrinks when being heated is used as the fixing means 48. However, the fixing means 48 is not limited to this example and various types of shrinkable tubes, an adhesive tape, an adhesive, or the like can be used as the fixing means 48.

In the illustrated example, the wire 42 is attached to neither the outer cylinder shaft 30 nor the inner cylinder shaft 35. However, the wire 42 is not limited to this example and may be attached to the outer cylinder shaft 30.

As illustrated in Fig. 3, even in a state of maximum relative movement of the inner cylinder shaft 35 to the outer cylinder shaft 30 toward the distal side in the longitudinal direction LD to stretch the balloon 25, the second temperature sensor 45 is located on the proximal end side of the balloon 25 and in the liquid passage LP between the outer cylinder shaft 30 and the inner cylinder shaft 35. According to this specific example, the second temperature sensor 45 can be located in the liquid passage LP without depending on a relative position of the inner cylinder shaft 35 to the outer cylinder shaft 30. Accordingly, the second temperature sensor 45 can stably be protected by the outer cylinder shaft 30 without depending on the relative position of the inner cylinder shaft 35 to the outer cylinder shaft 30.

Alternatively, unlike the illustrated example, the second temperature sensor 45 may be attached to the inner surface of the outer cylinder shaft 30. According to this specific example, the second temperature sensor 45 can stably be protected by the outer cylinder shaft 30 without depending on the relative position of the inner cylinder shaft 35 to the outer cylinder shaft 30.

Next, a description is made on the handle 50 connected to the catheter body 20 described above from the proximal side. The handle 50 is a portion held by the operator (practitioner) during use of the balloon catheter system 10. Thus, the handle 50 is preferably designed to allow the operator to easily hold and operate by hand. A material for forming the handle 50 is preferably a highly chemical-resistant material. For example, a polycarbonate or an acrylonitrile-butadiene-styrene copolymer (hereinafter, ABS resin) can be used.

The handle 50 illustrated in Fig. 1 has a first handle section 51 and a second handle section 52 that are mutually slidable. The first handle section (front handle section) 51 is connected to the outer cylinder shaft 30 of the catheter body 20. The second handle section (rear handle section) 52 is connected to the inner cylinder shaft 35 of the catheter body 20. By relative movement of the second handle section 52 to the first handle section 51, the inner cylinder shaft 35 can move relative to the outer cylinder shaft 30.

As illustrated in Fig. 1, the handle 50 also functions as a portion connecting other devices included in the balloon catheter system 10 to the balloon catheter 15.

First, a connector 56 extends from the second handle section 52. This connector 56 electrically connects the wire 42 of the catheter body 20 and the lead wire 47 of the temperature sensor 45 to the external controller 70. The connector 56 extends from one of plural branch sections 52a provided in the second handle section 52. When the wire 42 and the lead wire 47 are connected to the external device (controller 70) via the same handle section, as described above, the wire 42 and the lead wire 47 are preferably attached together to one of the outer cylinder shaft 30 and the inner cylinder shaft 35, particularly, to the shaft (the inner cylinder shaft 35 in the illustrated example) connected to the handle section. In this case, it is possible to further effectively avoid tangling and disconnection of the wire 42 and the lead wire 47.

The connector 56 is preferably configured to effectively prevent improper connection. In addition, the connector 56 preferably has an excellent water resistance property. The configuration of the connector 56 can be determined in consideration of convenience of the practitioner and design matters. Similar to the handle 50, the highly chemical-resistant material is preferably used as a material for forming the connector 56. As an example, the polycarbonate or the ABS resin is suited.

The connector 56 may have a high-conductivity metal pin therein. When being connected to this high-conductivity metal pin, each of the wire 42 and the lead wire 47 can electrically be connected to the controller 70 as high-frequency power supply means. However, the lead wire 47 of the second temperature sensor 45 may electrically be connected to a device other than the controller 70 as the high-frequency power supply means, such as a temperature indicator.

The material for the high-conductivity metal pin included in the connector 56 is not limited to any particular type as long as the material is a high-conductivity metal. Examples of the material for the high-conductivity metal pin included in the connector 56 includes copper, silver, gold, platinum, and the alloys thereof. In addition, an outer portion of the high-conductivity metal pin is preferably protected by an electrically-insulating and chemical-resistant material. Examples of the electrically-insulating and chemical-resistant material includes polysulfones, polyurethane-based polymers, polypropylenes, and polyvinyl chloride.

The second handle section 52 has branch sections 52b, 52c in addition to the branch sections 52a, to which the connector 56 is connected. These branch sections 52b, 52c function as a portion for supplying the liquid to the lumen as the internal space of the inner cylinder shaft 35 and a portion from which the guide wire inserted in the lumen of the inner cylinder shaft 35 extends. During the cardiac ablation treatment, it is common practice to discharge a minute amount of physiological saline, about 100 ml per hour, into the body through the lumen of the inner cylinder shaft 35. By discharging the physiological saline, it is possible to effectively prevent backflow of blood into the lumen of the inner cylinder shaft 35.

In addition, as illustrated in Fig. 1, an extension tube 57 extends from the first handle section 51. This extension tube 57 causes the liquid passage LP in the catheter body 20 to lead to an external supplier 74 and the external agitator 75. The extension tube 57 extends from a branch section 51a provided in the first handle section 51. The extension tube 57 is connected to the supplier 74 and the agitator 75 via a valve 58. In the illustrated example, the operation of the valve 58 enables selection of which of the supplier 74 and the agitator 75 is made to lead to the liquid passage LP. A three-way stopcock can be used as the valve 58.

Next, a description is made on the devices constituting the balloon catheter system 10 together with the balloon catheter 15 described so far, more specifically, the controller 70, the supplier 74, and the agitator 75.

The illustrated controller 70 is electrically connected to the heating member 40 composed of a coil electrode via the wire 42. The controller 70 has a high-frequency energization control section (not illustrated) for controlling high-frequency energization to the heating member 40. In the illustrated example, the high-frequency energization control section controls the high-frequency energization to the heating member 40, thereby adjusting output from the counter electrode 77. The high-frequency energization control section can control the high-frequency energization to the heating member 40 based on the temperature of the heating member 40 identified by the first temperature sensor and the surface temperature of the balloon 25 identified by a temperature calculation section described below, according to preset processing, or according to input from the operator.

In the case where the high-frequency energization is controlled by using only the temperature of the heating member 40 identified by the first temperature sensor, and, for example, in the case where a set temperature in the first temperature sensor is 70 °C, regardless of the surface temperature of the balloon 25 identified from the temperature acquired by the second temperature sensor 45, the high-frequency energization control section controls the high-frequency energization such that a high-frequency current is applied until the temperature of the heating member 40 identified by the first temperature sensor 31 reaches 70 °C.

Meanwhile, in the case where the high-frequency energization is controlled by using only the surface temperature of the balloon 25, which is identified from the temperature acquired by the second temperature sensor 45, and, for example, in the case where a set temperature for the surface temperature of the balloon 25 identified from the temperature acquired by the second temperature sensor 45 is 65 °C, regardless of the temperature of the heating member 40 identified by the first temperature sensor 31, the high-frequency energization control section controls the high-frequency energization such that the high-frequency current is applied until the surface temperature of the balloon 25 identified from the temperature acquired by the second temperature sensor 45 reaches 65 °C.

Furthermore, a description is made on control in a case where the high-frequency energization is controlled by using both the temperature of the heating member 40 identified by the first temperature sensor 31 and the surface temperature of the balloon 25 identified from the temperature acquired by the second temperature sensor 45.

For example, the set temperature for the temperature of the heating member 40 identified by the first temperature sensor 31 is 70 °C, and the set temperature for the surface temperature of the balloon 25 identified from the temperature acquired by the second temperature sensor 45 is 65 °C. In such a case, when the temperature acquired by the first temperature sensor 31 reaches 70 °C but the temperature acquired by the second temperature sensor 45 does not reach 65 °C, the temperature acquired by the second temperature sensor 45 is not increased to the set temperature. However, the high-frequency energization control section controls the high-frequency energization such that the high-frequency current is no longer applied. Meanwhile, when the temperature acquired by the first temperature sensor 31 does not reach 70 °C but the temperature acquired by the second temperature sensor 45 reaches 65 °C, the temperature acquired by the first temperature sensor 31 is not increased to the set temperature. However, the high-frequency energization control section controls the high-frequency energization such that the high-frequency current is no longer applied.

The controller 70 is electrically connected to the lead wire 47 of the first temperature sensor 31 and the lead wire 47 of the second temperature sensor 45. The controller 70 has the temperature calculation section (not illustrated) for calculating information on the temperature acquired by the second temperature sensor 45. The temperature calculation section calculates the liquid temperature in the liquid passage LP based on the information on the temperature acquired by the second temperature sensor 45, and further estimates the surface temperature of the balloon 25 based on the calculated liquid temperature. The temperature calculation section may show the identified surface temperature of the balloon 25 on a display 71.

Furthermore, the controller 70 has an agitator control section (not illustrated) for controlling the agitator 75. The agitator control section may show a control condition of the agitator 75 on the display 71.

The controller 70 is configured by hardware such as a CPU. One or more of the high-frequency energization control section, the temperature calculation section, and the agitator control section included in the controller 70 may be configured as separate hardware or may be supplied in part. At least a part of the controller 70 may be configured by software. A part of the controller 70 may be physically separated from the rest. In the controller 70, some of the components as the part may be able to cooperate with the other components by communication through a network. Alternatively, in the controller 70, some of the components may be devices, such as a cloud server or a cloud database, capable of communicating with the other components as the part via an external network.

Next, the supplier 74 is described. The supplier 74 supplies the liquid into the liquid passage LP. By injecting the liquid into the balloon 25 from the supplier 74 via the liquid passage LP, the balloon 25 can be inflated as illustrated in Fig. 2. Meanwhile, by discharging the liquid into the supplier 74 from the balloon 25 via the liquid passage LP, the balloon 25 can also be deflated. The liquid supplied into the liquid passage LP can typically be physiological saline. As illustrated in the drawing, a syringe can be used as the supplier 74. Alternatively, a pump or the like can be used as the supplier 74.

Next, the agitator 75 is described. The agitator 75 is provided to agitate the liquid in the balloon 25. By agitating the liquid in the balloon 25, it is possible to disperse or equalize the heat supplied into the balloon 25 and thereby adjust the surface temperature of the balloon 25. The agitator 75 repeatedly supplies the liquid into the liquid passage LP and discharges the liquid from the liquid passage LP. As an agitator 75, a pump selected from the group consisting of a roller pump, a diaphragm pump, a bellows pump, a vane pump, a centrifugal pump, and a pump composed of the combination of a piston and a cylinder can be selected.

A liquid supply amount to the liquid passage LP and a liquid discharge amount from the liquid passage LP can each be a fixed amount (for example, 5 ml or larger and 30 ml or smaller). In addition, the liquid may be repeatedly supplied to the liquid passage LP and discharged from the liquid passage LP in a fixed cycle (for example, one to five times per second). The liquid supply amount to the liquid passage LP and the liquid discharge amount from the liquid passage LP may be adjusted by a control signal from the agitator control section described above or by direct input from the operator. Similarly, the cycle of the liquid supply to the liquid passage LP and the liquid discharge from the liquid passage LP may be adjusted by the control signal from the agitator control section described above or by the direct input from the operator.

Next, an example of a method of using the balloon catheter system 10 configured as described so far is described.

First, the valve 58 is operated to cause the supplier 74 to lead to the liquid passage LP in the catheter body 20 via the handle 50. Thereafter, the supplier 74 is operated to inject the liquid into the liquid passage LP and fill the balloon, the liquid passage LP, and the extension tube 57 with the liquid. Next, the inner cylinder shaft 35 is made to move relative to the outer cylinder shaft 30 toward the distal end (tip) side in the longitudinal direction LD, thereby stretching the balloon 25 as illustrated in Fig. 3. At this time, by operating the first handle section 51 and the second handle section 52 of the handle 50, the outer cylinder shaft 30 and the inner cylinder shaft 35 can move relative to each other. Then, the catheter body 20 with the stretched balloon 25 is inserted into the body.

When the distal end of the catheter body 20 is guided near the target site (affected area), the inner cylinder shaft 35 is made to move relative to the outer cylinder shaft 30 toward the proximal (base end) side in the longitudinal direction LD, thereby loosening the balloon 25. Next, the valve 58 is operated to cause the supplier 74 to lead to the liquid passage LP in the catheter body 20 via the handle 50. Thereafter, the supplier 74 is operated to inject the liquid into the liquid passage LP and inflate the balloon 25 with the liquid as illustrated in Fig. 2.

Next, the valve 58 is operated to disconnect the supplier 74 from the liquid passage LP and causes the agitator 75 to lead to the liquid passage LP. The agitator 75 is controlled by the control signal from the agitator control section of the controller 70. The agitator 75 repeatedly supplies the fixed amount of the liquid to the liquid passage LP and discharges the fixed amount of the liquid from the liquid passage LP with a fixed cycle. In this way, injection of the fixed amount of the liquid into the balloon 25 from the liquid passage LP and suctioning of the fixed amount of the liquid into the liquid passage LP from the balloon 25 are repeated in the fixed cycle. As a result, the liquid in the balloon 25 is agitated.

In addition, the heating member 40 is controlled by the high-frequency energization control section of the controller 70 to adjust the liquid temperature in the balloon 25. More specifically, the controller 70 performs the high-frequency energization between the heating member 40 composed of the coil electrode and the counter electrode 77 arranged outside the patient body. As a result, a high-frequency current is generated between the heating member 40 and the counter electrode 77. Here, by reducing the longitudinal width of the coil in the coil electrode to be less than the width of the counter electrode, current density around the heating member 40 is increased, thereby heating the surrounding liquid and contrast agent by Joule heating.

The liquid in the balloon 25 is agitated while being heated as described so far. Then, the balloon 25 containing the heated liquid is pressed against the target site to ablate the target site. During the ablation, the second temperature sensor 45 arranged in the liquid passage LP obtains the information on the liquid temperature in the liquid passage LP. The acquired information is calculated by the temperature calculation section of the controller 70. In particular, the temperature calculation section not only identifies the liquid temperature in the area where the second temperature sensor 45 is arranged, but can also identify the surface temperature of the balloon 25 with a high degree of accuracy as described below. The surface temperature of the balloon 25, which is identified by the temperature calculation section with a high degree of accuracy, is shown on the display 71, for example.

That is, by using this balloon catheter system 10, the operator can perform the ablation treatment while preventing excessive heating of the portion whose temperature becomes the highest in the balloon catheter, and grasping the accurate surface temperature of the balloon 25 at all times.

When the ablation of the target site is completed, energy supply to the heating member 40 is stopped. In addition, the valve 58 is operated to cause the supplier 74 to lead to the liquid passage LP in the catheter body 20 via the handle 50 and disconnects the agitator 75 from the liquid passage LP. Then, the liquid is discharged from the liquid passage LP by using the supplier 74 to deflate the balloon 25. Next, the second handle section 52 is operated to stretch the deflated balloon 25 as illustrated in Fig. 3. Then, the catheter body 20 with the stretched balloon 25 is removed from the body. This terminates the procedure using the balloon catheter system 10.

Fig. 6 illustrates a second embodiment of the distal end portion of the balloon catheter. The proximal end of the balloon 25 is connected to the side surface of the outer cylinder shaft 30, and the distal end (tip) of the outer cylinder shaft 30 projects into the balloon 25. The outer cylinder shaft 30 has a tubular section 30b having a large diameter on the proximal side, a tubular section 30c having a small diameter on the distal side, and a tapered transition section 30a connecting the tubular section having the large diameter and the tubular section having the small diameter therebetween.

In this case, the extending section 36, which is the section extending into the balloon 25, is formed by the tubular section 30c having the small diameter and a part of the tapered transition section 30a, which are provided in the outer cylinder shaft 30. The tapered transition section 30a has a hole 44, through which the conductive wire runs and which serves as the liquid passage. The heating member 40 composed of the coil electrode is formed by a conductive wire wound around the tubular section 30c having the small diameter.

In the outer cylinder shaft 30 in the second embodiment, the outer diameter of the tubular section 30b having the large diameter is preferably 3.0 mm or larger and 4.0 mm or smaller, and the inner diameter of the tubular section 30b having the large diameter is preferably 2.5 mm or larger and 3.5 mm or smaller. The outer diameter of the tubular section 30c having the small diameter is preferably 1.5 mm or larger and 3.0 mm or smaller, and the inner diameter of the tubular section 30c having the small diameter is preferably 1.5 mm or larger and 2.0 mm or smaller. The tapered transition section 30a of the outer cylinder shaft 30 has one or more holes 44, and the diameter of the hole 44 is preferably 0.5 mm² or larger and 2 mm² or smaller.

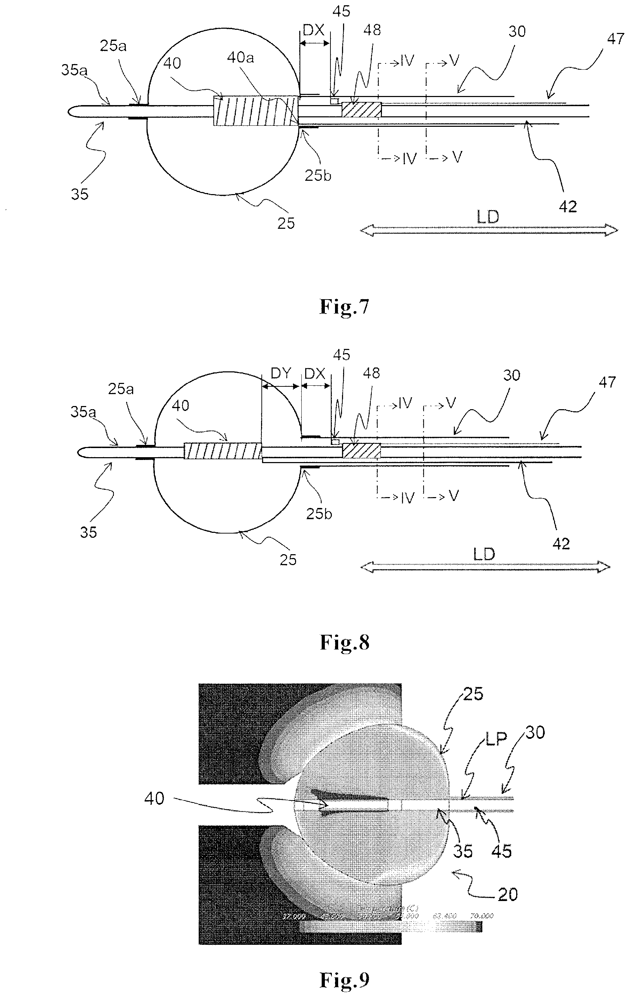

Fig. 7 illustrates a third embodiment of the distal end portion of the balloon catheter. In this embodiment, the outer cylinder shaft 30 has a single inner diameter and a single outer diameter, and the outer cylinder shaft 30 connected to the proximal end of the balloon 25 does not project into the balloon 25. The heating member 40 composed of the coil electrode is formed by a self-supporting conductive wire formed in a coil shape. The proximal end (proximal end) 25b of the balloon 25 is fixed to the side surface of the outer cylinder shaft 30, and the proximal end (proximal end) 40a of the heating member 40 is fixed to the distal end (tip) of the outer cylinder shaft 30. The heating member 40 was formed by coiling the wire 42 on a mandrel and fixing both ends of the coil by soldering, welding, or the like.

The outer diameter of the coil electrode in the third embodiment is preferably 3.0 mm or larger and 4.0 mm or smaller. The inner diameter of the coil electrode is preferably 2.5 mm or larger and 3.5 mm or smaller.

### EXAMPLES

Next, a detailed description is made on the relationship between the temperature of the heating member 40 detected by the first temperature sensor 31 and the surface temperature of the balloon 25, which can be detected by the second temperature sensor 45, by using examples and a comparative example.

### <Example 1>

In order to manufacture the balloon catheter 15 according to Example 1, the balloon 25 having a diameter of 30 mm and a thickness of 20 µm and made of polyurethane was fabricated by blow molding.

A polyurethane tube having an outer diameter of 3.6 mm, an inner diameter of 3.0 mm, and a total length of 1000 mm was used as the tubular intermediate member 46. A polyamide tube having an outer diameter of 1.6 mm, an inner diameter of 1.2 mm, and a total length of 1100 mm was used as the inner cylinder shaft 35. Furthermore, the fixed member 43 was connected to the distal end (tip) of the tubular intermediate member 46 to form the outer cylinder shaft 30. The tubular section 43c having the small diameter in the fixed member 43 was set to have an outer diameter of 2.0 mm and an inner diameter of 1.7 mm, and the tubular section 43b having the large diameter was set to have an outer diameter of 2.9 mm and an inner diameter of 2.7 mm. The tapered transition section 43a, which connected the tubular section having the large diameter and the tubular section having the small diameter, was formed with four holes, each of which had a hole diameter of 1 mm².

The electrically insulating protective coating applied to the wire 42 and the lead wire 47 was partially peeled off, and the wire 42 was then coiled around the fixed member 43 while clipping the lead wire 47 to form the single electrode having an electrode length of 13 mm, the heating member 40 composed of the coil electrode, and the first temperature sensor 43. On the inner cylinder shaft, the wire 42 and the lead wire 47 are welded near the heating member 40 to form the second temperature sensor 45.

The inner cylinder shaft 35 was slidably inserted into the lumen of the outer cylinder shaft 30. Then, the distal end 25a of the balloon 25 was fixed to the distal end 35a of the inner cylinder shaft 35. The distal end 30a of the outer cylinder shaft 30 was inserted into the balloon 25 from the proximal end 25b of the balloon 25, and the proximal end 25b of the balloon 25 was fixed to the proximal portion of the outer cylinder shaft 30 from the distal end 30a.

A polycarbonate handle 50 was provided at rear ends of the outer cylinder shaft 30 and the inner cylinder shaft 35. The handle 50 was constituted by the first handle section (front handle section) 51 connected to the outer cylinder shaft 30 and the second handle section (rear handle section) 52 connected to the inner cylinder shaft 35. With this constitution, by a sliding operation of the second handle section 52 against the first handle section 51, the inner cylinder shaft 35 slid inside the outer cylinder shaft 30, thereby being able to deform the balloon 25.

### <Example 2>

In this example, the outer cylinder shaft 30 was a stepped shaft whose diameter varies through a taper. As dimensions of the stepped shaft, the tubular section 30c having the small diameter extending into the balloon 25 had an outer diameter of 2.0 mm and an inner diameter of 1.7 mm, and the tubular section 30b having the large diameter had an outer diameter of 3.6 mm and an inner diameter of 3.0 mm. In addition, a balloon catheter 15 was fabricated in the same manner as in Example 1 except that the four holes having a hole diameter of 1 mm² at the connections were formed in the portion connected to the balloon 25.

### <Example 3>

The electrically insulating protective coating applied to the wire 42 and the lead wire 47 was partially peeled off, and the wire 42 was then coiled around a mandrel while clipping the lead wire 47, and both of the ends of the coil were soldered to form a single electrode having an electrode length of 13 mm, the heating member 40 composed of the coil electrode, and the first temperature sensor 43. Thereafter, the mandrel was removed, and the wire 42 and the lead wire 47 were fixed to the distal end 30a of the outer cylinder shaft 30 by using an adhesive. In this way, the balloon catheter 15 was fabricated in the same manner as in Example 1 except that the length between the heating member 40 and the distal end 30a of the outer cylinder shaft 30 was made constant even when the outer cylinder shaft 30 entered the balloon 25. In this example, the outer cylinder shaft 30 does not have the extending section, which extends into the balloon, at the distal end.

### <Comparative Example>

With reference to Fig. 8, the balloon catheter 15 was fabricated in the same manner as in Example 1 except that the heating member 40 was formed on the inner cylinder shaft 35 without using the fixed member 43.

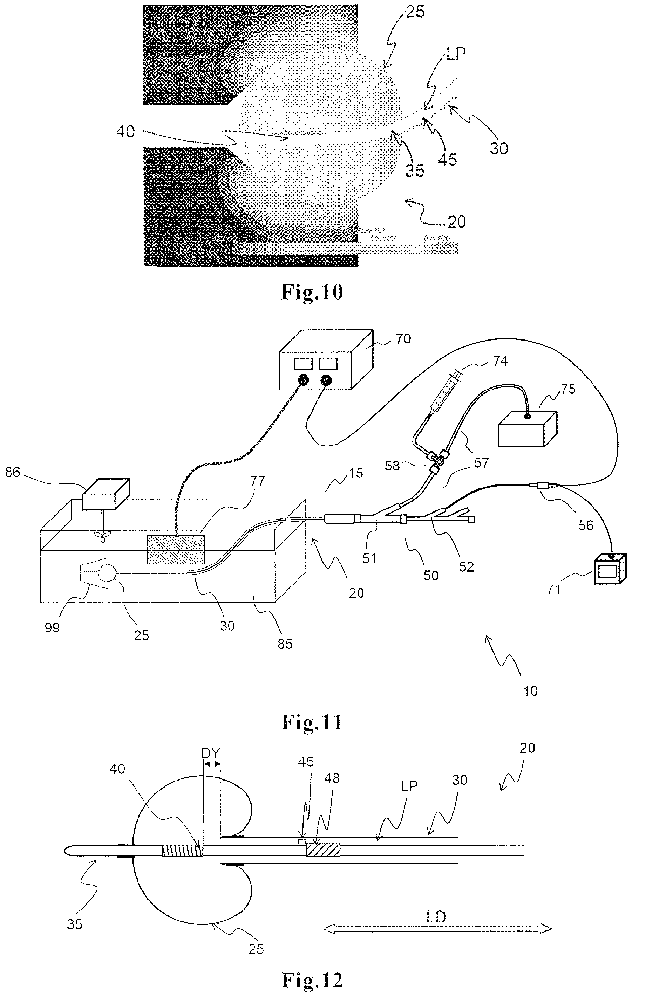

First, Figs. 9 and 10 each shows a simulation result of the temperature distribution in the balloon 25 by the CAE (computer added engineering) in the comparative example. Fig. 6 shows the simulation result in a state where the balloon 25 is pressed against the target site along the longitudinal direction LD (hereafter also simply referred to as a "coaxial state"). In the example shown in Fig. 9, the outer cylinder shaft 30 is aligned substantially in line with the inner cylinder shaft 35 and the heating member 40 in the balloon 25. Meanwhile, Fig. 10 shows the simulation result in a state where the balloon 25 is pressed against the target site in a direction inclined with respect to the longitudinal direction LD (hereafter also simply referred to as a "non-coaxial state"). In the comparative example illustrated in Fig. 8, the outer cylinder shaft 30 is largely inclined with respect to the inner cylinder shaft 35 and the heating member 40 in the balloon 25 as shown in Fig. 10.

According to this simulation result, even when the liquid in the balloon 25 is agitated by using the agitator 75, a temperature gradient occurs to the liquid in the balloon 25 due to the arrangement of the heating member 40. When the heating member 40 composed of the coil electrode is used, this temperature distribution shows a similar tendency to that of current density distribution. The temperature distribution of 5 °C or higher occurs in the balloon 25 in the coaxial state shown in Fig. 6, and the temperature distribution of 10 °C or higher occurs in the non-coaxial state illustrated in Fig. 10. Thus, it is understood that the temperature distribution in the balloon 25 changes depending on the pressing state of the balloon 25 against the target site and other factors.

Fig. 11 illustrates the balloon catheter system 10 used in an experiment conducted to check the relationship between the temperature of the heating member 40 detected by the first temperature sensor 31 and the surface temperature of the balloon 25 capable of being detected by the second temperature sensor 45. In this experiment, an actual measured value of the surface temperature of the balloon 25 was compared to an actual measured value of the liquid temperature in the liquid passage LP, which was identified based on the information acquired by the temperature sensor 45. In this experiment, the above-described balloon catheter system 10 illustrated in Fig. 1 was used.

As illustrated in Fig. 11, the detection result of the first temperature sensor 31 was retrieved by the temperature calculation section of the controller 70. The high-frequency energization control section received the calculation result from the temperature calculation section and controlled the high-frequency energization to the heating member 40 based on the information acquired by the first temperature sensor 31.

In this experiment, the ablation treatment was performed on a pseudo living body 99 that mimicked a pulmonary vein of the left atrium in the human body. The pseudo living body 99 was immersed in the physiological saline kept in a tank 85. During the experiment, a tank agitator 86 was used to agitate the physiological saline in the tank 85. The counter electrode 77 generating the high-frequency current with the heating member 40 composed of the coil electrode in the catheter body 20 was arranged on a side wall of the tank 85. The physiological saline in the tank 85 was prepared by dissolving 0.9 wt% of salt (sodium chloride) in water.

The liquid supplied from the supplier 74 into the liquid passage LP and the balloon 25 was prepared by further mixing the physiological saline containing 0.9 wt% of salt (sodium chloride) dissolved in water with a radiographic contrast agent. The injection amount of the liquid into the balloon 25 was set at two levels of 10 mL and 20 mL, which were frequently used in the actual ablation treatment. Omnipaque (registered trademark, manufactured by GE HealthCare Pharma) was used as the contrast agent mixed in the liquid.

The high-frequency energization control section of the controller 70 controlled the high-frequency energization such that the temperature of the heating member 40 was 70 °C. The drive power was set at 150 W.

In the experiment, the injection amount of the liquid into the balloon 25 was changed at the two levels of 10 ml and 20 ml, and a contact state of the balloon 25 was changed at two levels of the coaxial state and the non-coaxial state. Furthermore, two levels of 5 mm and 9 mm were selected for the length DY between the heating member 40 and the proximal end 25b of the balloon 25 in order to replicate the following situation. When the balloon catheter 15 was pressed against the target site during the ablation, the outer cylinder shaft 30 advances toward the distal side in the longitudinal direction LD, and the balloon rear end portion 25b was pushed in as illustrated in Fig. 12, thereby reducing the length DY. Moreover, in order to replicate a state where the balloon catheter 15 was not pressed against the target site, 13 mm of the length DY was selected as a standard, and three levels of 5 mm, 9 mm, and 13 mm were used for the length DY.

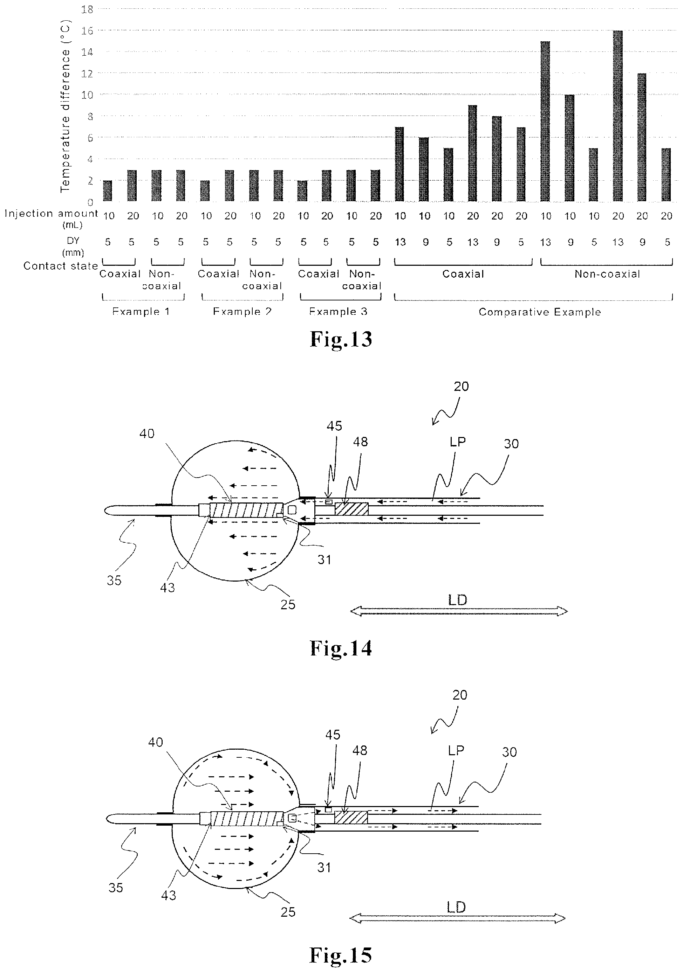

Under a total of 24 different conditions in which the liquid injection amount, the balloon contact state, and the length DY between the heating member and the proximal end of the balloon were changed in each of Examples 1 to 3 and the comparative example, simulation tests using the balloon catheter system 10 for the pseudo living body 99 were conducted. Fig. 13 shows a difference between the temperature detected by the first temperature sensor 31 and the measured temperature value by the second temperature sensor 45 for each condition in each experiment. Fig. 13 shows an average value after a lapse of a period in which the surface temperature of the balloon 25 is sufficiently stabilized after the start of the high-frequency energization, more specifically, in a period of 150 seconds to 200 seconds after the start of the energization.

The following findings were obtained from the experimental results shown in Fig. 13. When the heating member 40 is fixed to the inner cylinder shaft 35 as in the comparative example, the balloon surface temperature is greatly affected by the liquid injection amount, the balloon contact state, and the length DY between the heating member and the proximal end of the balloon, which makes it necessary to increase the temperature of the heating member 40 in order to keep the balloon surface temperature constant. Meanwhile, when the heating member 40 is fixed to the outer cylinder shaft 30 as in Examples, it was found that the balloon surface temperature is less likely to be affected by the liquid injection amount and the balloon contact state and is not changed by the length DY between the heating member and the proximal end of the balloon.

It is assumed that such a result was obtained due to the following reason. In the case where the heating member 40 is fixed only to the outer cylinder shaft 30, as illustrated in Figs. 14 to 17, an agitation flow in the balloon 25 is not easily changed even when the balloon contact state or the like is changed, and the positional relationship between the heating member 40 and the outer cylinder shaft 30 is constant at all times. Thus, a heating member cooling effect by the liquid supplied from the liquid passage LP into the balloon 25 is always the same.

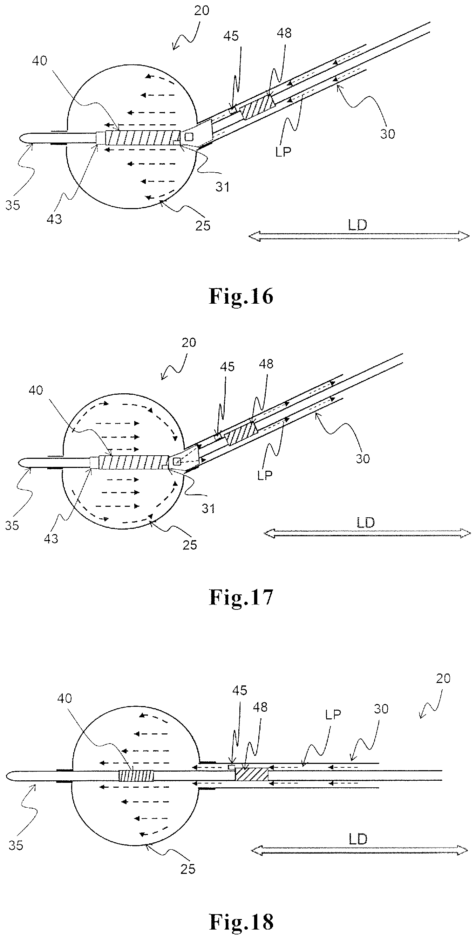

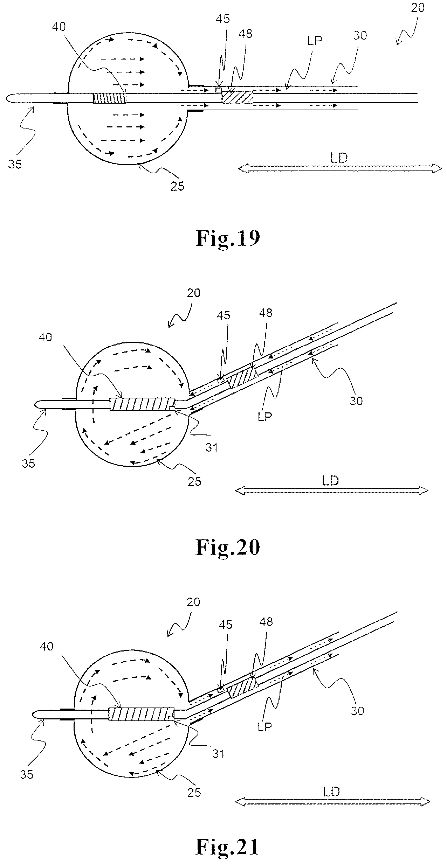

Meanwhile, it is assumed that, in the case where the heating member 40 is fixed only to the inner cylinder shaft 35, as illustrated in Figs. 18 to 21, the agitation flow in the balloon 25 is easily changed by a change in the balloon contact state or the like, and thus the heating member cooling effect by the liquid supplied from the liquid passage LP into the balloon 25 is not constant.

In order to safely keep the balloon surface temperature constant, it is considered that the stabilization of the cooling effect for the heating member 40 by the liquid supplied from the liquid passage LP into the balloon 25 is extremely important. In this way, it is possible to provide the balloon catheter capable of stably and efficiently diffusing the liquid heated in the balloon without being affected by the catheter operation and capable of safely obtaining the stable balloon surface temperature (the surface temperature of the balloon is not changed between the coaxial state and the non-coaxial state) and to provide the catheter system having such a balloon catheter.

The embodiment has been described by using the plural examples. However, these examples are not intended to limit the embodiment. The above-described embodiment can be implemented in various other examples, and various types of omission, replacement, modification, addition, and the like can be made without departing from the gist thereof.

For example, in the above-described example of the embodiment, the single liquid passage LP is provided between the outer cylinder shaft 30 and the inner cylinder shaft 35, the liquid is supplied into the balloon 25 via this single liquid passage LP, and the liquid is discharged from the balloon 25.

However, the embodiment is not limited to this example, and two or more liquid passages LP may be provided between the outer cylinder shaft 30 and the inner cylinder shaft 35. In this modified example, two or more of the liquid passages LP may include a supply liquid passage for supplying the liquid into the balloon 25 and a discharge liquid passage for discharging the liquid from the balloon 25.

Also, in this modified example, it is possible to provide the safe ablation catheter capable of keeping the constant relationship between the temperature of the heating member 40 detected by the first temperature sensor 31 and the surface temperature of the balloon 25 detected by the second temperature sensor 45 by fixing the heating member 40 only to the outer cylinder shaft 30.

### INDUSTRIAL APPLICABILITY

The present invention can be used for the balloon catheter system and the balloon catheter for treating arrhythmia caused by atrial fibrillation, endometriosis, cancer, and the like.

### DESCRIPTION OF SYMBOLS

10: balloon catheter system, 15: balloon catheter, 20: catheter body, 25: balloon, 25a: distal end, 25b: proximal end, 30: outer cylinder shaft, 30a tapered transition section, 30b: tubular section having large diameter, 30c: tubular section having small diameter, 31: first temperature sensor, 35: inner cylinder shaft, 40: heating member, 42: wire, 43: fixed member, 43a: tapered transition section, 43b: tubular section having large diameter, 43c: tubular section having small diameter, 44: hole, 45: second temperature sensor, 46: tubular intermediate member, 47: lead wire, 50: handle, 70: controller, 75: agitator, 77: counter electrode, 99: pseudo living body, LD: longitudinal direction, LP: liquid passage, DX: length between proximal end of balloon and second temperature sensor, DY: length between heating member and proximal end of balloon

## Claims

1. A balloon catheter, comprising:
a balloon;
an outer cylinder shaft connected to a proximal end of the balloon;
an inner cylinder shalt passing through the outer cylinder shaft and connected to a distal end of the balloon;
a heating member for heating a liquid in the balloon;
a first temperature sensor fixed to an end of the heating member; and
a liquid passage formed between the outer cylinder shaft and the inner cylinder shaft and leading into the balloon,
wherein the balloon can be stretched or loosened by relative movement of the inner cylinder shaft to the outer cylinder shaft, and
the heating member is arranged in the balloon and fixed only to the outer cylinder shaft.

2. The balloon catheter of claim 1,
wherein the outer cylinder shaft has an extending section extending into the balloon, and
the heating member is fixed to the extending section.

3. A balloon catheter system, comprising:
the balloon catheter of claim 1 or 2;
a supplier for supplying the liquid to the liquid passage;
an agitator for agitating the liquid in the balloon by repeatedly supplying the liquid to the liquid passage and discharging the liquid from the liquid passage; and
a heater electrically connected to the heating member and applying electrical energy to the heating member,
wherein the heater applies the electrical energy to the heating member based on information on a temperature acquired by the first temperature sensor.
